# EUROPÄISCHE PATENTANMELDUNG

(11) **EP 4 141 009 A1**
(43) Veröffentlichungstag der Anmeldung: **01.03.2023**
(21) Anmeldenummer: 21193854.3
(22) Anmeldetag: 30.08.2021
(51) Int. Cl.: C07D 497/04, A01N 47/00, C10G 70/04, B01D 3/00

(54) **VERFAHREN ZUR HERSTELLUNG VON MISCHUNGEN ENTHALTEND 2 (2 HYDROXYETHYL)-PIPERIDINYL-CARBAMIDSÄURE-SEK.-BUTYLESTER**

(71) Anmelder: Saltigo GmbH, 51369 Leverkusen (DE)
(72) Erfinder: LANGER, Reinhard, 47918 Tönisvorst (DE)
(74) Vertreter: Matzke, Michael

(57) **Zusammenfassung**

Die Erfindung umfasst ein verbessertes Verfahren zur Herstellung von Mischungen enthaltend 2-(2-Hydroxyethyl)-piperidinyl-carbamidsäure-sek.-butylester aus einem Rohprodukt durch thermische Behandlung mit kurzen Verweilzeiten, Vorrichtungen für dieses Verfahren, Verwendung solcher Vorrichtungen für solche Verfahren, sowie die aus dem Verfahren erhältlichen erfindungsgemäßen Mischungen.

## Beschreibung

Die Erfindung umfasst ein verbessertes Verfahren zur Herstellung von Mischungen enthaltend 2-(2-Hydroxyethyl)-piperidinyl-carbamidsäure-sek.-butylester aus einem Rohprodukt durch thermische Behandlung mit kurzen Verweilzeiten, Vorrichtungen für dieses Verfahren, Verwendung solcher Vorrichtungen für solche Verfahren, sowie die aus dem Verfahren erhältlichen erfindungsgemäßen Mischungen.

2-(2-Hydroxyethyl)-piperidinyl-carbamidsäure-sek.-butylester (auch als 1-Piperidin-carboxylsäure-2-(2-hydroxyethyl)-1-methylpropylester bezeichnet; CAS-Nr. 119515-38-7, INN: Icaridin, Picaridin, Saltidin^{®}, Vertriebsprodukt Saltidin^{®} der Saltigo GmbH, Leverkusen, Deutschland), weist die folgende Struktur der Formel (I), 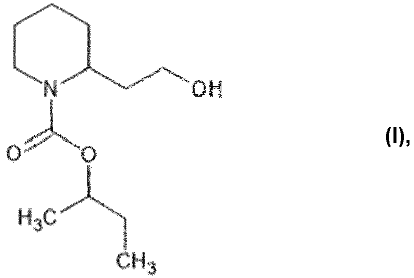 auf. Das Molekül enthält 2 Stereozentren. In Formel (I) sind erfindungsgemäß alle einzelnen 4 möglichen Stereoisomere, sowie deren beliebige Mischungen und die Racemate, also die Mischungen der einzelnen oder der beiden möglichen Enantiomerenpaare zu gleichen Teilen, mit umfasst.

Diese Verbindung ist ein hochwirksames und verträgliches Insektenrepellent, das gegen Mücken, Zecken und in verschiedenen Formulierungen für die Anwendung auf menschlicher oder tierischer Haut kommerziell erhältlich ist.

Die Herstellung von 2-(2-Hydroxyethyl)-piperidinyl-carbamidsäure-sek.-butylester ist beispielsweise in EP 0 537 534 A1 beschrieben, nach dem 2-(2-Hydroxyethyl)-piperidinyl-carbamidsäure-sek.-butylester in Ausbeuten von 89 Prozent der Theorie (bezogen auf 2-(2-Hydroxyethyl)piperidin) erhalten wird. In der letzten Stufe erfolgt die chemische Umsetzung von 2-(2-Hydroxyethyl)piperidin mit Chlorameisensäure-sek.-butylester in Gegenwart von nicht mit Wasser mischbaren Lösungsmitteln und Base. Bei der Reaktion entsteht das gewünschte Produkt neben z.B. Natriumchlorid und organischen Nebenkomponenten, die teilweise gefärbt sind und/oder einen unangenehmen Geruch aufweisen können. So beschreibt die EP 0 537 534 A1, dass Rohprodukte aus dieser Reaktion oft verunreinigt seien und daher durch Destillation oder durch Chromatographie gereinigt werden müssten, was neben dem erhöhten Aufwand auch zu beträchtlichen Ausbeuteverlusten führen kann. Weiterhin ist darin beschrieben, dass als Folge der Hauptnebenreaktion Produkte gebildet werden, die sich aus einer konkurrierenden Reaktion des Chlorameisensaeureesters mit der Hydroxylgruppe aus dem 2-(2-Hydroxyethyl)piperidin ableiten. So ist nach den üblichen bekannten Herstellungsverfahren die Verbindung der Formel (II),

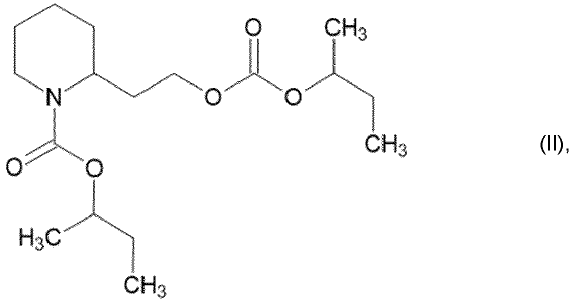

in einem Gehalt von ca. 1 Gew.% und mehr enthalten. Weiterhin weisen die nach den üblichen Verfahren hergestellten Mischungen, die mehr als 90 Gew.% Verbindung der Formel (I) enthalten, eine Hazen-Farbzahl von mehr als 40 auf.

Bei der destillativen Reinigung können auch bei niedrigen Drucken Zersetzungsreaktionen auftreten, deren übelriechende Produkte das Destillat verunreinigen. Daher konnte die Destillation als Aufreinigungsmethode für 2-(2-Hydroxyethyl)-piperidinyl-carbamidsäure-sek.-butylester im industriellen Maßstab bisher nicht erfolgreich eingesetzt werden.

Üblicherweise wird das nach der Reaktion erhaltene Reaktionsgemisch daher vorteilhafterweise wässrig aufgearbeitet, wobei gegebenenfalls noch vorhandene Base durch Zugabe von Säure neutralisiert werden muss. Danach erfolgt üblicherweise die Abtrennung der organischen Phase, die das gewünschte Produkt sowie organische Nebenkomponenten und das bei der Reaktion eingesetzte nicht mit Wasser mischbare organische Lösungsmittel, beispielsweise Hexan oder Toluol, enthält. Es hat sich herausgestellt, dass im industriellen Maßstab, in dem das Produkt in Batchgrößen von mehr als 1.000 kg hergestellt wird, mehrere Wäschen der so erhaltenen organische Phase mit Wasser und / oder Säure durchgeführt werden müssen, um ein für die Herstellung von Insekten-Repellent-Formulierungen geeignetes Produkt, das die Anforderung an Reinheit, Farbe und olfaktorische Qualität erfüllt, zu erhalten. Nach den Wäschen wird üblicherweise das Lösungsmittel von der organischen Phase im Vakuum abdestilliert, wobei das Produkt sowie die gesamten bzw. ein Teil der organischen und ggf. anorganischen Nebenkomponenten bzw. Verunreinigungen im Sumpf verbleiben. Der Sumpf wird dann aus dem Reaktor entnommen, filtriert und stellt dann das fertige Endprodukt dar. In dem bisher großtechnisch durchgeführten Verfahren wird das Produkt 2-(2-Hydroxyethyl)-piperidinyl-carbamidsäure-sek.-butylester auf diese Weise in Ausbeuten von ca. 90 Prozent der Theorie bezogen auf 2-(2-Hydroxyethyl)piperidin in Reinheiten von 97,0 bis 98,5 Gewichtsprozent erhalten. Es weist üblicherweise eine Hazen-Farbzahl von 40 oder mehr sowie einen leichten, charakteristischen unangenehmen Geruch auf. Obwohl nach diesem derzeit besten Verfahren 2-(2-Hydroxyethyl)-piperidinyl-carbamidsäure-sek.-butylester in sehr guten Ausbeuten und Reinheiten auch im industriellen Maßstab erhältlich ist, weisen die Verfahren nach dem Stand der Technik dennoch den Nachteil auf, dass die Aufarbeitung des Rohproduktes sehr aufwändig und energieintensiv ist, viele Abfälle und damit hohe Kosten erzeugt.

Im Stand der Technik sind jedoch auch Synthesen von 2-(2-Hydroxyethyl)-piperidinyl-carbamidsäure-sek.-butylester bekannt, in denen sich nach der Reaktion von (2-(2-Hydroxyethyl)piperidin mit Chlorameisensäure-sek.-butylester in Gegenwart von Lösungsmittel mehrere extraktive Wäschen der organischen Phase und als letzter Schritt eine Destillation des Produkts im Vakuum anschließt.

So beschreibt CN 102167681 A1 die Umsetzung von 2-(2-Hydroxyethyl)-piperidin mit Chlorameisensäure-sek.-butylester in Gegenwart von Lösungsmitten wie Dichloromethan, Chloroform, Benzol, Toluol, Tetrahydrofuran, n-Hexan, Cyclohexan oder Methylcyclohexan als Lösungsmittel. Als Base werden organische Basen wie 2-(2-Hydroxyethyl)piperidin, Triethylamin, Pyridin, oder Trimethylamin eingesetzt. Die organische Phase, die das Rohprodukt enthält, wurde mehrmals mit Wasser und Kochsalzlösung gewaschen. Anschließend wurde die organische Phase mit Natriumsulfat oder Magnesiumsulfat getrocknet, filtriert und das Lösungsmittel abdestilliert. Der Rückstand wurde dann destilliert, wobei das Produkt als Destillat mit einem Siedepunkt von 140 bis 152 °C bei 5 mm Hg (6,67 hPa) erhalten wurde. Bei Batchgrößen von ca. 20 kg Produkt wurde das Produkt nach diesem Verfahren in Ausbeuten von 84 bis 87 Prozent der Theorie und mit einem Gehalt von 97,5 bis 98,0 Flächenprozenten (über Gaschromatographie ermittelt) erhalten.

Eigene Versuche haben jedoch ergeben (siehe Beispiele), dass bei der Destillation eines Rohproduktes, das ebenfalls nach mehreren wässrigen Wäschen erhalten wurde, bei der Destillation im Vakuum bei 20 hPa und ca. 175 °C Sumpftemperatur eine starke Zersetzung des Produkts auftrat, bei der 2-Butanol abgespalten wurde und das cyclische Carbamat des 2-(2-Hydroxyethyl)-piperidins der Formel (III), 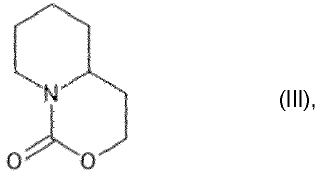 entstand, wobei das Butanol nicht im Kondensator, in dem üblicherweise das Produkt der Formel (I) kondensiert, sondern erst in einer nachgeschalteten Kühlfalle kondensierte und das Carbamat im Sumpf verblieb.

Da im großtechnischen Maßstab eine Batch-Destillation bei unter 10 hPa technisch praktisch nicht durchführbar ist, scheidet dafür eine Aufarbeitung nach der CN 102167681 A1 aus.

Außerdem weisen die nach bisherigen industriellen Maßstäben erhältlichen Produkte, die mehr als 90 Gew.% Verbindung der Formel (I) enthalten, einen leichten, aber unangenehmen Geruch auf, der in Insekten-Repellent-Formulierungen stören kann und gegebenenfalls durch Duftstoffe maskiert werden muss.

Damit bestand nach wie vor das technische Problem, ein Verfahren zur Herstellung von 2-(2-Hydroxyethyl)-piperidinyl-carbamidsäure-sek.-butylester bereitzustellen, das die Nachteile der Verfahren des Standes der Technik nicht aufweist. Es bestand daher das Bedürfnis nach einem verbesserten Verfahren, nach dem 2-(2-Hydroxyethyl)-piperidinyl-carbamidsäure-sek.-butylester in einer zumindest gleich guten Ausbeute und Reinheit oder sogar in einer höheren Ausbeute und/oder mit weniger unerwünschten Nebenkomponenten und/oder besseren farblichen und/oder olfaktorischen Qualität im industriellen Maßstab erhältlich ist, aber weniger Zeit und Energie verbraucht und weniger Abfälle erzeugt und damit die technische Aufgabe, ein solches verbessertes Verfahren bereitzustellen.

Überraschenderweise wurde jetzt ein verbessertes Verfahren zur Herstellung einer Mischung gefunden, enthaltend als Komponenten von 98,8 bis 100,0 Gew.%, bevorzugt von 99,0 bis 99,9 Gew.%, Verbindung der Formel (I), 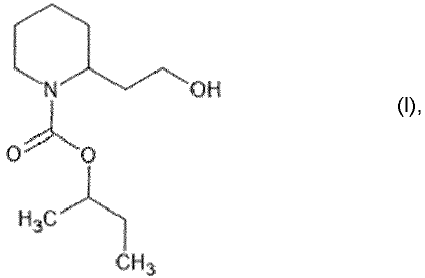 von 0,00 bis 0,70 Gew.%, bevorzugt von 0,0001 bis 0,20 Gew.%, Verbindung der Formel (II),

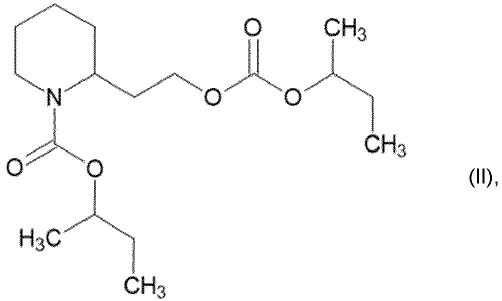

sowie weitere Bestandteile, wobei sich die Gewichtsprozente der Komponenten zu 100 Gew.% addieren, wobei die Mischung eine Hazen-Farbzahl von 0 bis 15 gemessen nach Methode DIN EN ISO 6271 aufweist,

### ausgehend von einen Rohprodukt enthaltend

∘ von 94 bis 99,0 Gew.% Verbindung der Formel (I) und
∘ von 0,50 bis 5,0 Gew.% Gew.% Verbindung der Formel (II),
wobei zumindest eine Fraktion A (Destillat, erfindungsgemäße Mischung) von einer Fraktion B (Sumpf) und einer gasförmigen Fraktion C abgetrennt wird, indem
a) das Rohprodukt als flüssige Phase derart thermisch behandelt wird, sodass ein Gasstrom G enthaltend Fraktion A und Fraktion C erzeugt wird, und die Fraktion B als flüssige Phase verbleibt, und
b) gleichzeitig und / oder nachfolgend Fraktion B flüssig aus dem Verfahren ausgeschleust wird, und
c) gleichzeitig und / oder nachfolgend Fraktion A aus Gasstrom G kondensiert und aus dem Verfahren ausgeschleust wird, wobei die erfindungsgemäße Mischung erhalten wird, und
d) gegebenenfalls gleichzeitig und / oder nachfolgend die Fraktion C kondensiert und aus dem Verfahren ausgeschleust wird,
wobei die Verweilzeit der flüssigen Phase während der thermischen Behandlung, bevorzugt die Verweilzeit der flüssigen Phase enthaltend ein Rohprodukt und/oder Fraktion B oder deren beliebigen Mischungen, von 1 bis 900 Sekunden, bevorzugt von 10 bis 600 Sekunden, besonders bevorzugt von 10 bis 300 Sekunden, beträgt.

Bevorzugt können mit dem erfindungsgemäßen Verfahren auch Mischungen hergestellt werden, die als Komponenten von 98,8 bis 100,0 Gew.%, bevorzugt von 99,0 bis 99,9 Gew.%, Verbindung der Formel (I), von 0,00 bis 0,70 Gew.%, bevorzugt von 0,0001 bis 0,20 Gew.%, Verbindung der Formel (II), von 0,0001 bis 2,0 Gew.% Verbindung der Formel (III), sowie weitere Bestandteile enthalten,
wobei sich die Gewichtsprozente der Komponenten zu 100 Gew.% addieren,
wobei die Mischung eine Hazen-Farbzahl von 0 bis 15 gemessen nach Methode DIN EN ISO 6271 aufweist.

In dem erfindungsgemäßen Verfahren wird ein Rohprodukt eingesetzt, das aus dem chemischen Herstellungsverfahren und nachfolgenden, üblicherweise extraktiven, Reinigungsschritten resultiert.

Bevorzugt wird in das erfindungsgemäße Verfahren ein Rohprodukt eingesetzt, das von 94,0 bis 99,0 Gew.% Verbindung der Formel (I) sowie von 0,50 bis 5,0 Gew.% Verbindung der Formel (II) enthält und das eine Hazen-Farbzahl von mindestens 40 gemessen nach Methode DIN-ISO 6271 aufweist.

Beispielsweise wird das Rohprodukt nach einen Verfahren hergestellt umfassend
i. Umsetzung von 2-Hydroxyethylpiperidin mit Chlorameisensäure-sek.-butylester in Gegenwart oder Abwesenheit zumindest einer Base und in Gegenwart oder Abwesenheit zumindest eines Lösungsmittels, wobei ein Reaktionsgemisch erhalten wird, das von 35 bis 65 Gew.% Verbindung der Formel (I) enthält, und
ii. nachfolgend, gegebenenfalls unter Vermischung, Zugabe von Säure und/oder Wasser zu dem Reaktionsgemisch aus Schritt i., wobei ein zweiphasiges Reaktionsgemisch umfassend eine organische und eine wässrige Phase entsteht, und
iii. nachfolgend gegebenenfalls Trennung der organischen Phase von der wässrigen Phase des Reaktionsgemisches aus Schritt ii., und
iv. nachfolgend gegebenenfalls Wäsche der organischen Phase aus Schritt iii. mit wässriger Säure, und
v. nachfolgend gegebenenfalls Trocknung der organischen Phase aus Schritt iv., und
vi. nachfolgend gegebenenfalls Abtrennung des zumindest einen Lösungsmittels von der organischen Phase aus Schritt v., wobei das Rohprodukt erhalten wird.

In einer bevorzugten Ausführungsform kann dieses Rohprodukt in das erfindungsgemäße Verfahren eingesetzt werden.

In dem erfindungsgemäßen Verfahren erfolgt eine Trennung von drei unterschiedlichen Fraktionen, wobei Fraktion A die erfindungsgemäßen Mischungen darstellt, eine höher siedende Fraktion B eine Mischung darstellt, die üblicherweise von 40 bis 95 Gew.% Verbindung der Formel (I), sowie von 1,5 bis 50 Gew.% Verbindung der Formal (II) aufweist, sowie eine niedriger siedende Fraktion C eine Mischung darstellt, die üblicherweise von 0,0 bis 0,5 Gew.% Verbindung der Formel (I), aufweist und im Wesentlichen Wasser, Butanol und Toluol enthält, je nach Qualität des Einsatzmaterials. In dem erfindungsgemäßen Verfahren wird dabei das flüssige Rohprodukt als flüssige Phase thermisch durch geeignete technische Mittel behandelt. Dabei ist für das erfindungsgemäße Verfahren wesentlich, dass die Verweilzeit der flüssigen Phase während der thermischen Behandlung, bevorzugt die Verweilzeit der flüssigen Phase enthaltend ein Rohprodukt und/oder Fraktion B oder deren beliebigen Mischungen, von 1 bis 900 Sekunden, bevorzugt von 10 bis 600 Sekunden, besonders bevorzugt von 10 bis 300 Sekunden beträgt.

Anschaulich beschreibt die Verweilzeit die Zeit vom Beginn der thermischen Behandlung des Rohprodukts, d.h. vom Beginn des Eintritts des Rohprodukts in den Bereich, in dem die thermische Behandlung erfolgt, über die Verarmung des Rohprodukts an Gasstrom G innerhalb des Bereichs, in dem die thermische Behandlung erfolgt, unter Bildung der dann vorliegenden Fraktion B, bis zum Ende der thermischen Behandlung, d.h. bis zu dem Zeitpunkt, an dem die Fraktion B den Bereich, in dem die thermische Behandlung erfolgt, wieder verlässt.

Bevorzugt erfolgt die thermische Behandlung des Rohprodukts dadurch, dass das Rohprodukt in Kontakt mit einer festen erhitzen Oberfläche gebracht wird.

Die Verweilzeit der flüssigen Phase während der thermischen Behandlung wird erfindungsgemäß bei den Temperaturen und Drucken und dem Wärmeeintrag gemessen, bei denen auch das erfindungsgemäße Verfahren durchgeführt wird. Dem Fachmann sind dafür die entsprechenden Methoden bekannt. Beispielweise kann die Verweilzeit mit der sogenannten Tracer-Methode ermittelt werden. Bei diesen Messungen werden typischerweise Verteilungskurven der Verweilzeit (residence time distribution, RTD) erhalten. Die mittlere Verweilzeit lässt sich dann mit üblichen Methoden rechnerisch ermitteln. Die mittlere Verweilzeit kann auch mit vereinfachten Methoden genügend genau ermittelt werden. Beispielsweise ist die mittlere Verweilzeit T, beispielsweise gemessen in Sekunden, bei einem kontinuierlichen Verfahren, das sich im Gleichgewichtszustand befindet, der Quotient V / R aus dem Volumen V der flüssigen Phase innerhalb des Raums, beispielsweise gemessen in Kubikzentimeter, in dem die thermische Behandlung erfolgt und der Zuflussrate R der flüssigen Phase, beispielsweise gemessen in Kubikzentimeter pro Sekunde, in den Raum, in dem die thermische Behandlung stattfindet. Erfindungsgemäß ist die Verweilzeit die aus der gemessenen Verweilzeitverteilung berechnete und/oder mit vereinfachten Methoden bestimmte mittlere Verweilzeit.

Das erfindungsgemäße Verfahren schließt Destillationsverfahren aus, in denen die flüssige Phase in einem Prozess derart thermisch behandelt wird, dass die gesamte ursprüngliche flüssige Phase in einem Volumen erhitzt wird, und daraus die flüchtigen Anteile verdampft werden. Bei diesen sogenannten Batch-Destillationen ist die Verweilzeit abhängig von der Destillationsdauer. Bei einer gleichmäßigen Destillatentnahme, beispielsweise gemessen in Kubikzentimeter pro Sekunde, entspricht die mittlere Verweilzeit der flüssigen Phase in einer Batchdestillation die Hälfte der Destillationsdauer.

Während des bevorzugten erfindungsgemäßen Verfahrens weist die feste Oberfläche eine Temperatur auf, die ausreicht, um einen Gasstrom G zu erzeugen, der Fraktion A und Fraktion C enthält. Fraktion B geht an der erhitzten festen Oberfläche nicht in die Gasphase über, sondern wird über technische Mittel als flüssiger Film von der festen erhitzten Oberfläche wegtransportiert, in der Regel in ein für Fraktion B vorgesehenes Behältnis. Gasstrom G hat bei Umgebungsdruck einen Siedepunkt von ca. 330 °C, bei 20 hPa einen Siedepunkt von 178 bis 182 °C, bei 35 hPa von 194 bis 198 °C, und weist bei 20 hPa gemessen am Kopf des Verdampfungsraums üblicherweise eine Temperatur von 170 bis 180 °C auf.

Bei dem erfindungsgemäßen Verfahren erfolgt die Erzeugung des Gasstroms G bevorzugt bei einer Temperatur von 120 bis 200 °C, bevorzugt von 120 bis 185 °C, besonders bevorzugt von 120 bis 175 °C und einem Druck von 1 bis 35 hPa, bevorzugt von 1 bis 21 hPa, besonders bevorzugt von 2 bis 15 hPa. Bei höheren Drücken und den daraus erforderlichen höheren Temperaturen zur Erzeugung des Gasstroms G nimmt die Zersetzung der Verbindung der Formel (I) zu. Bevorzugt wird bei den genannten Drucken die Temperatur für die erhitzte feste Oberfläche so gewählt, dass sie maximal 40 °C, bevorzugt maximal 30 °C, über dem Siedepunkt der Fraktion A, also der erfindungsgemäßen Mischung bei dem jeweiligen Druck liegt.

In dem erfindungsgemäßen Verfahren wird aus Gasstrom G Fraktion A kondensiert, üblicherweise über ein geeignetes technisches Mittel, bevorzugt über einen Kondensator. Dabei wird bevorzugt die Kondensation bei einer Temperatur von 80 bis 100 °C bei einem Druck von 2 bis 5 hPa, oder bei entsprechenden Siedetemperatur-Druckverhältnissen, durchgeführt. Fraktion C verbleibt während dieser Kondensation in der Gasphase.

In einer Ausführungsform des erfindungsgemäßen Verfahrens kann Fraktion C bei einer Temperatur von -10 bis 30 °C bei einem Druck von 2 bis 5 hPa, oder bei entsprechenden Siedetemperatur-Druckverhältnissen separat kondensiert werden. In einer weiteren Ausführungsform des erfindungsgemäßen Verfahrens kann Fraktion C in der Gasphase verbleiben und beispielsweise durch Verbrennung entsorgt werden.

Das Verfahren erfolgt bevorzugt kontinuierlich. Dabei wird kontinuierlich das Rohprodukts thermisch behandelt, gleichzeitig Fraktion B flüssig aus dem Verfahren ausgeschleust, gleichzeitig Gasstrom G erzeugt und von dem technischen Mittel, mit dem es thermisch behandelt wird, bevorzugt von der festen erhitzten Oberfläche, wegtransportiert. Gleichzeitig wird dann Fraktion A und gegebenenfalls Fraktion C kondensiert. Damit stehen diese unterschiedlichen Prozesse während des erfindungsgemäßen Verfahrens in einem Gleichgewicht. In diesem Gleichgewicht ist die Masse des dem Verfahren zugegebenen Rohprodukts immer im Wesentlichen identisch mit der Summe der Massen an Fraktion A, Fraktion B und Fraktion C, die aus dem Verfahren ausgeschleust werden. "Im Wesentlichen" bedeutet in diesem Zusammenhang, dass die technisch bedingten Schwankungen der Massenströme pro individueller Zeiteinheit damit inbegriffen sind.

Ein weiterer Vorteil einer bevorzugten Ausführungsform des erfindungsgemäßen Verfahrens ist, dass dem Rohprodukt zu keinem Zeitpunkt des Verfahrens ein Schiebemittel zugefügt wird. Dadurch werden weitere Abfälle vermieden.

Die Erfindung umfasst ebenfalls eine erfindungsgemäße Vorrichtung umfassend zumindest eine Verdampfungseinheit (1), Behälter für Fraktion B (3), einen Kondensator (5) und eine Pumpe (8).

Bevorzugt umfasst die erfindungsgemäße Vorrichtung zusätzlich eine Kolonne (4), eine kommunizierende Leitung (11) zwischen Verdampfungseinheit (1) und Kolonne (4), einen Rückflussteiler (6), und einen Kondensator (7).

Weiterhin bevorzugt umfasst die erfindungsgemäße Vorrichtung zusätzlich Einlass für Rohprodukt (21), kommunizierende Leitung (41) zwischen Kolonne (4) und Kondensator (5), kommunizierende Leitung (51) zwischen Kondensator (5) und Kondensator (7), Auslass für Fraktion C (72), kommunizierende Leitung (61) zwischen Kondensator (5) und Rückflussteiler (6), kommunizierende Leitung (62) zwischen Kolonne (4) und Rückflussteiler (6), und Auslass für Fraktion A (63).

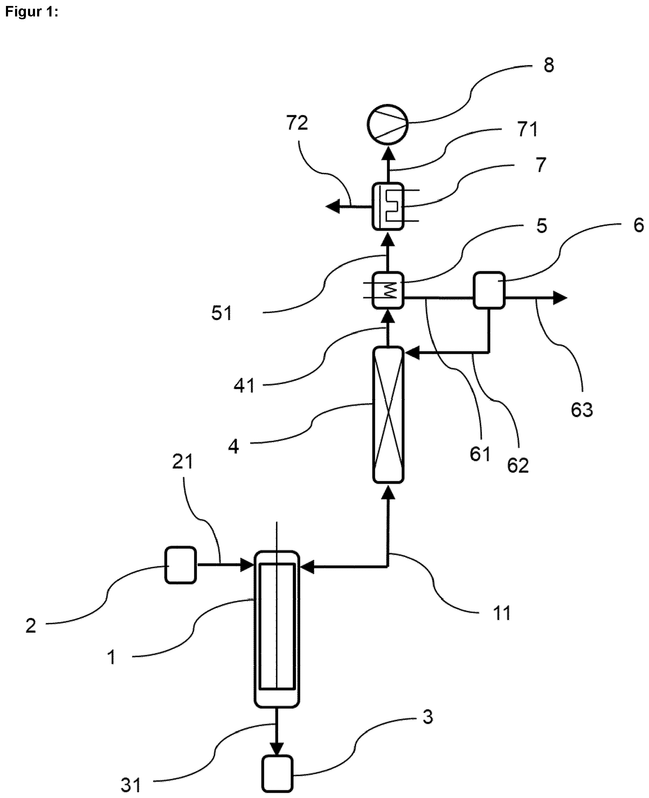

In Figur 1 ist die Darstellung einer besonders bevorzugten erfindungsgemäßen Vorrichtung wiedergegeben.

In Figur 1 bedeuten:
- 1: Verdampfungseinheit
- 2: Behältnis für Rohprodukt
- 3: Behälter für Fraktion B
- 4: Kolonne
- 5: Kondensator
- 6: Rückflussteiler
- 7: Kondensator
- 8: Pumpe
- 11: Auslass für Gasstrom G, kommunizierende Leitung zwischen 1 und 4
- 21: Einlass für Rohprodukt
- 31: Auslass für Fraktion B
- 41: Kommunizierende Leitung zwischen 4 und 5
- 51: Kommunizierende Leitung zwischen 5 und 7
- 61: Kommunizierende Leitung zwischen 5 und 6
- 62: Kommunizierende Leitung zwischen 6 und 4
- 63: Auslass für Fraktion A
- 71: Kommunizierende Leitung zwischen 6 und 7
- 72: Auslass für Fraktion C

In einer Ausführungsform wird das erfindungsgemäßen Verfahren in einer Vorrichtung durchgeführt wird, die zumindest
- eine Verdampfungseinheit (evaporation unit) (1), Einlass für Rohprodukt (21) und Auslass für Fraktion B (31), umfasst,
   ∘ wobei die Verdampfungseinheit (evaporation unit) (1) zumindest
   ∘ einen beheizbaren Gehäusemantel, welcher einen sich in axialer Richtung erstreckenden rotationssymmetrischen Verdampfungsraum (evaporation space) umschließt, und
   ∘ eine in dem Verdampfungsraum angeordnete und sich koaxial erstreckende, antreibbare Rotorwelle zur Erzeugung eines Rohproduktfilms auf der Innenfläche des Gehäusemantels und zur Förderung des Materials in Richtung vom Einlass für Rohprodukt (21) zum Auslass für Fraktion B (31) hin, wobei die Rotorwelle einen zentralen Rotorwellenkörper und auf dessen Umfang angeordnete Rotorelemente umfasst, deren radial äußerstes Ende einen Abstand zur Innenfläche des Gehäusemantels aufweist,
- Kondensator (5), Rückflussteiler (6), Auslass für Fraktion A (63), und bevorzugt Kolonne (4), umfasst,
   ∘ wobei in Schritt a) das Rohprodukt derart thermisch behandelt wird, dass das Rohprodukt über den Einlass (21) in den Verdampfungsraum der Verdampfungseinheit (1) eingetragen und auf der Innenseite des Gehäusemantels ein flüssiger Film des Rohproduktes erzeugt wird, wodurch der flüssige Film des Rohproduktes auf eine Temperatur erhitzt wird, sodass zumindest ein Teil der Fraktion A und der Fraktion C in den gasförmigen Zustand überführt und als Gasstrom G aus der Verdampfungseinheit (1), bevorzugt über den Auslass (11), ausgetragen wird, wobei das Verhältnis der Massenströme zwischen Fraktion B und Gasstrom G von 1 : 20 bis 1 : 5 beträgt, und
   ∘ in Schritt b) die Fraktion B über den Auslass (31) aus der Verdampfungseinheit (1) ausgetragen wird, und
   ∘ in Schritt c) Gasstrom G aus der Verdampfungseinheit (1), bevorzugt über Kolonne (4), in den Kondensator (5) überführt wird, wobei Fraktion A kondensiert und über den Rückflussteiler (6) und Auslass (63) erhalten wird.

Vorzugsweise umfasst die erfindungsgemäße Vorrichtung zusätzlich einen Kondensator (7), in dem gegebenenfalls in Schritt d) Fraktion C kondensiert wird und gegebenenfalls Fraktion C über Auslass für Fraktion C (72) ausgeschleust wird.

Bevorzugt ist Verdampfungseinheit 1 ein Dünnschichtverdampfer oder ein Fallfilmverdampfer, Besonders bevorzugt ist Verdampfungseinheit 1 ein Dünnschichtverdampfer.

Üblicherweise ist die Verdampfungseinheit 1 zylindrisch ausgebildet und weist einen äußeren und einen inneren Gehäusemantel auf. Der Gehäusemantel kann einwandig oder doppelwandig ausgebildet sein. Vorteilhaft ist der Gehäusemantel wegen der Temperaturdifferenz zwischen den inneren und dem äußeren Teilen der Verdampfungseinheit 1 doppelwandig ausgelegt. In dem Zwischenraum können dann Isolierungselemente und/oder Heizelemente eingebaut sein, die eine möglichst genaue Temperierung der Innenfläche des Gehäusemantels oder des inneren Gehäusemantels ermöglicht. "Innen" bedeutet in diesem Zusammenhang immer die Seite einer Fläche, die in Richtung des Mittelpunktes des Querschnitts des zylindrischen Verdampfungsraumes gerichtet ist. Koaxial zum rotationssymmetrischen Verdampfungsraum, der seitlich von der Innenfläche des Gehäusemantels begrenzt wird, ist ein durch einen Motor angetriebener Rotor angeordnet, der aus einer Rotorachse und daran angebrachten Rotorelementen besteht. Die Rotorelemente dehnen sich radial vom Mittelpunkt der Rotorachse in Richtung Innenfläche des Gehäusemantels aus. Es gibt starre und dynamische Rotorelemente.

Starre Rotorelemente sind beispielsweise Starrflügel-Rotoren, Radialwischer-Rotoren oder Wischerflügel-Rotoren. Hier endet entweder ein unflexibles Segment in einem kurzen Abstand zur Innenfläche des Gehäusemantels, oder flexible Segmente, beispielsweise bewegliche Flügel oder Wischer, werden durch die Elastizität des Materials und/oder durch die Zentrifugalkraft, die durch die Drehung des Rotors entsteht, in Richtung der Innenfläche des Gehäusemantels gedrückt. Bei Abwesenheit eines Produkts könnten die Enden dieser flexiblen Elemente die Innenfläche des Gehäusemantels berühren. Bei dynamischen Rotoren werden beispielsweise bewegliche Rollen benutzt, die an beweglichen Armen sitzen, die ihrerseits wieder mit der Rotorachse verbunden sind. Durch die Zentrifugalkraft, die durch die Drehung des Rotors entsteht, werden die Rollen in Richtung der Innenfläche des Gehäusemantels gedrückt und könnten so, ohne dass sich Produkt im Verdampfungsraum befindet, die Innenfläche des Gehäusemantels berühren. Wird aber in den Verdampfungsraum ein Produkt mit verdampfbaren Anteilen eingebracht, wird dieses Produkt mittels geeigneten technischen Mitteln in Richtung des oberen Teils der Innenfläche des Gehäusemantels dirigiert und dann von den Endsegmenten des Rotors entweder mittels des vorhandenen Spaltes zwischen dem äußersten Ende eines starren Flügels, oder mittels der flexiblen Endsegmente der Wischer oder Rollen als Film auf der Innenfläche des Gehäusemantels verteilt. Dabei wird die Dicke des Films durch ein Wechselspiel von zumindest der Viskosität des Produktes, der Art und Geschwindigkeit des Rotors und der Rotorelemente gesteuert.

In einer bevorzugte Ausführungsform des erfindungsgemäßen Verfahrens wird das Rohprodukt als flüssiger Film, der eine Filmdicke von 0,001 bis 2 mm, bevorzugt von 0,005 bis 1 mm aufweist, thermisch behandelt.

In einer weiteren bevorzugten Ausführungsform des erfindungsgemäßen Verfahrens wird das Rohprodukt als flüssiger Film, der eine Filmdicke von 0,001 bis 2 mm, bevorzugt von 0,005 bis 1 mm, aufweist, derart thermisch behandelt, indem es mit einer festen erhitzten Oberfläche in Kontakt gebracht.

In einer weiteren bevorzugten Ausführungsform des erfindungsgemäßen Verfahrens wird das Rohprodukt als flüssiger Film, der eine Filmdicke von 0,001 bis 2 mm, bevorzugt von 0,005 bis 1 mm, aufweist, in der Verdampfungseinheit (1) thermisch behandelt. In dieser weiteren bevorzugten Ausführungsform bildet die Innenfläche des Gehäusemantels der Verdampfungseinheit (1) die erhitzte feste Oberfläche, mit der das Rohprodukt in Kontakt gebracht wird.

Die Filmdicke ergibt sich aus den technischen Parametern der dafür verwendeten Vorrichtung sowie aus den dabei eingesetzten technischen Mitteln. Dies wird weiter unten detaillierter beschrieben. Der flüssige Film wird in direkten Kontakt mit einer festen erhitzten Oberfläche gebracht. Die feste erhitzte Oberfläche kann beispielsweise eine Innenwand eines Dünnschichtverdampfers oder eines Fallfilmverdampfers darstellen. Bevorzugt ist die feste Oberfläche eine Innenwand eines Dünnschichtverdampfers.

Die Bestimmung der Filmdicke erfolgt beispielsweise derart, dass bei einer definierten Zuflussrate eines spezifischen Produkts bei Normaldruck mit der festen erhitzten Oberfläche, die die Temperatur aufweist, die bei der Durchführung des bevorzugten erfindungsgemäßen Verfahrens angewandt wird, in Kontakt gebracht wird. Dabei wird die Zeit gemessen, die für das gesamte Benetzen der festen erhitzten Oberfläche unter Erzeugung eines Films erforderlich ist. Aus dem Produkt T x R der gemessenen Zeit T, bspw. 120 Sekunden, und der Zuflussrate R, bspw. 300 cm³/ 3600 sec., ergibt sich das Volumen V des Films, bspw. 10 cm³, der über der gesamten Fläche der festen erhitzten Oberfläche erzeugt wurde. Da die Fläche der festen erhitzten Oberfläche A, bspw. aus dessen geometrischen Daten berechnet werden kann, lässt sich die Filmdicke F als Quotient V/A, d.h. Volumen des Films geteilt durch Fläche der festen erhitzten Oberfläche A, berechnen. In dem gegebenen Beispiel beträgt die Fläche A 1000 cm² und damit die Filmdicke 0,01 cm. Ohne dass Anteile des Produkts verdampfen, beträgt die mittlere Verweilzeit des Produkts an der festen erhitzten Oberfläche in diesem Beispiel 120 sec. Andererseits kann auch die mittlere Verweilzeit bestimmt werden, wenn die Fläche A bekannt ist und das Volumen gemessen wird, das für die Benetzung der gesamtem Fläche A bei einer Zuflussrate R erforderlich ist.

In dem bevorzugten erfindungsgemäßen Verfahren beträgt die Verweilzeit der flüssigen Komponenten, also Rohprodukt und/oder Fraktion B, oder deren beliebigen Mischungen, an der festen erhitzten Oberfläche bevorzugt von 1 bis 900 Sekunden, bevorzugt von 10 bis 600 Sekunden, besonders bevorzugt von 10 bis 300 Sekunden.

Für das bevorzugte erfindungsgemäße Verfahren wird bei der Verwendung einer Verdampfungseinheit (1), beispielsweise von Dünnschichtverdampfern, mit Filmdicken von 0,001 bis 2 mm, bevorzugt von 0,001 bis 1 mm, gearbeitet. Die Bestimmung der Filmdicke erfolgt hier beispielsweise derart, dass bei einer definierten Zuflussrate eines spezifischen Produkts, das in der Verdampfungseinheit 1, beispielsweise in einem Dünnschichtverdampfer, verarbeitet werden soll, einer definierten Rotorgeschwindigkeit und bei einer definierten Temperatur der Innenfläche des Gehäusemantels der Verdampfungseinheit 1, jedoch bei Normaldruck die Zeit gemessen wird, die für das gesamte Benetzen der Innenfläche des Gehäusemantels zur Erzeugung eines erforderlich ist. Aus dem Produkt T x R der gemessenen Zeit T, bspw. 50 Sekunden, und der Zuflussrate R, bspw. 300 cm³/ 3600 sec., ergibt sich das Volumen V des erzeugten Films, bspw. 5 cm³, der durch den Rotor über die gesamte Innenfläche des Gehäusemantels erzeugt wurde. Da die Fläche der Innenfläche des Gehäusemantels A, bspw. aus dessen geometrischen Daten berechnet werden kann, lässt sich die Filmdicke F als Quotient V/A, d.h. Volumen des Films geteilt durch Fläche der Innenfläche des Gehäusemantels A, berechnen. In dem gegebenen Beispiel beträgt die Filmdicke 0,05 mm. Die mittlere Verweilzeit des der flüssigen Phase in der Verdampfungseinheit 1 beträgt in diesem Beispiel 60 sec.

In dem bevorzugten erfindungsgemäßen Verfahren beträgt die Verweilzeit der flüssigen Komponenten, also Rohprodukt und/oder Fraktion B, oder deren beliebigen Mischungen, an der festen erhitzten Oberfläche von 1 bis 900 Sekunden, bevorzugt von 10 bis 600 Sekunden, besonders bevorzugt von 10 bis 300 Sekunden.

In dem bevorzugten erfindungsgemäßen Verfahren, das eine Verdampfungseinheit 1 verwendet, wird die Temperatur für die Innenfläche des Gehäusemantels bevorzugt so gewählt, dass sie maximal 40 °C, bevorzugt maximal 30 °C, über dem Siedepunkt der Fraktion A bei dem jeweiligen Druck liegt. Ebenfalls bevorzugt erfolgt in Schritt a) das Überführen der Gasstroms G in die Gasphase durch Inkontaktbringen mit der Innenfläche des Gehäusemantels der Verdampfungseinheit (1) bei einer Temperatur von 120 bis 200 °C, bevorzugt von 120 bis 185 °C, besonders bevorzugt von 120 bis 175 °C und einem Druck von 1 bis 35 hPa, bevorzugt von 1 bis 21 hPa, besonders bevorzugt von 2 bis 15 hPa.

Das Rohprodukt wird dabei bevorzugt durch Einlass **21** in den oberen Teil der Verdampfungseinheit **1** über geeignete technische Mittel direkt auf den oberen Teil der erhitzten Innenfläche des Gehäusemantels der Verdampfungseinheit **1** geleitet und beginnt durch die Schwerkraft nach unten zu fließen, bis die rotierenden Enden der Segmente der Rotorelemente beginnen, das Rohprodukt als flüssigen Film gleichmäßig auf der erhitzten Innenfläche des Gehäusemantels der Verdampfungseinheit **1** zu verteilen. Der Fachmann kann dabei die für die Verdampfungseinheit **1** optimale Wischgeschwindigkeit des Rotors durch einfache Versuche ermitteln. Eine zu geringe Wischgeschwindigkeit führt zu einer unvollständigen Verteilung des flüssigen Films und ggf. zur Bildung von Tropfen, die schneller durch die Verdampfungseinheit **1** fließen als der flüssige Film. Über einen optimalen Wert der Wischgeschwindigkeit hinaus bringt eine weitere Steigerung der Wischgeschwindigkeit keine weitere Verbesserung des flüssigen Films. Durch nachfließendes Rohprodukt, durch die Schwerkraft und durch die mechanische Kraft der rotierenden Enden der Rotorelemente wandert der flüssige Film des Rohprodukts langsam zum unteren Ende der erhitzten Innenfläche des Gehäusemantels der Verdampfungseinheit **1.** Durch den Kontakt des flüssigen Films des Rohprodukts mit der erhitzten Innenfläche des Gehäusemantels der Verdampfungseinheit **1** geht ein Teil des Rohprodukts als Gasstrom G in die Gasphase über, der Fraktion A und Fraktion C enthält. Fraktion B geht an der erhitzten Innenfläche des Gehäusemantels der Verdampfungseinheit **1** nicht in die Gasphase über, sondern wird flüssig durch die Schwerkraft und/oder durch die Bewegung der Rotorelemente kontinuierlich in Richtung des Bodens der Verdampfungseinheit **1** transportiert, an dem sich Auslass **31** für Fraktion B befindet. Gasstrom G wird durch den durch die Kondensation erzeugten Druckgradienten in Richtung des oberen Teils der Verdampfungseinheit **1** geleitet, an dem sich Auslass **11** für Gasstrom G befindet. Auslass **11** ist bevorzugt kommunizierend mit Kolonne **4** verbunden. Kolonne **4** ist besonders bevorzugt eine Kolonne, die Kolonneneinbauten, Füllkörperschüttungen oder feste Packungen aufweist. Bevorzugt weist die Kolonne **4** von 1 bis 20, bevorzugt von 5 bis 15, besonders bevorzugt von 8 bis 12, theoretische Böden auf.

Innerhalb von Kolonne **4** besteht während des bevorzugten erfindungsgemäßen Verfahrens ein Dampf-Kondensat-Gleichwicht mit einem nach oben gerichteten Konzentrationsgradienten. In diesem Konzentrationsgradienten nimmt zum Boden der Kolonne der Anteil an höher siedenden Komponenten zu. Ein Teil des Gasstroms G verlässt in dieser bevorzugten Ausführungsform gasförmig kontinuierlich den Kopf der Kolonne **4** und gelangt über die Leitung **41,** die mit Kondensator **5** kommunizierend verbunden ist, in den Kondensator **5.** Kondensator **5** weist üblicherweise eine Innentemperatur von 50 bis 110 °C, bevorzugt von 70 bis 110°C, auf, sodass Fraktion A kondensiert, während Fraktion C den oberen Teil des Kondensators **5** gasförmig über Auslass **51** verlässt. Die kondensierte Fraktion A verlässt den Kondensator **5,** bevorzugt über Auslass **61,** der mit einer Leitung verbunden ist, die bevorzugt mit einem Rückflussteiler **6** kommunizierend verbunden ist. Ein Teil der kondensierten Fraktion A verlässt dabei den Rückflussteiler **6** über Auslass **63,** während ein weiterer Anteil der kondensierten Fraktion A den Rückflussteiler **6** über Leitung **62,** die kommunizierend mit dem oberen Teil der Kolonne **4** verbunden ist, wieder in den oberen Teil der Kolonne durch Schwerkraft zurückfließt.

In einer besonders bevorzugten Ausführungsform des erfindungsgemäßen Verfahrens ist im Rückflussteiler **6** der Rückfluss so eingestellt, dass das Verhältnis der Massenströme, die über die Leitung **62** und Leitung **63** den Rückflussteiler **6** verlassen, von 1 : 10 bis 1 : 1, bevorzugt von 1 : 5 bis 1 : 2, beträgt. Beispielsweise verlässt den Rückflussteiler **6** bei einem Massenstromverhältnis von 1 : 10 die zehnfache Masse an kondensierter Fraktion A über Leitung **63** im Vergleich zu der Masse an Fraktion A, die den Rückflussteiler **6** über Leitung **62** verlässt, und in Kolonne **4** zurückgeführt wird. Über Leitung **63** wird mit Fraktion A die erfindungsgemäße Mischung erhalten, die von 98,8 bis 100,0 Gew.%, bevorzugt von 99,0 bis 99,9 Gew.%, Verbindung der Formel (I), von 0,00 bis 0,70 Gew.%, bevorzugt von 0,0001 bis 0,20 Gew.%, Verbindung der Formel (II) enthält und die eine Hazen-Farbzahl von 0 bis 15 gemessen nach Methode DIN EN ISO 6271 aufweist.

Der Massenstrom an kondensierter Fraktion A, der über die Leitung **62** wieder in die Kolonne zurückfließt, fließt auch teilweise wieder in die Verdampfungseinheit **1** zurück.

Bei einer bevorzugten Ausführungsform des erfindungsgemäßen Verfahrens erfolgt die Erzeugung des Gasstroms G bei Drücken von 1 bis 30 hPa. Gasstrom G weist üblicherweise bei einem Druck von 20 hPa eine Temperatur von 170 bis 180 °C auf. Bei höheren Drücken und den daraus erforderlichen höheren Temperaturen zur Erzeugung des Gasstroms G nimmt die Zersetzung der Verbindung der Formel (I) zu. Bevorzugt wird bei den genannten Drücken die Temperatur für die Innenwand der Verdampfungseinheit **1** so gewählt, dass sie maximal 40 °C, bevorzugt maximal 30 °C, über dem Siedepunkt der Fraktion A bei dem jeweiligen Druck liegt. Ebenfalls bevorzugt wird die erhitzte feste Oberfläche auf Temperaturen von 120 bis 200 °C, bevorzugt von 120 bis 185 °C, besonders bevorzugt von 120 bis 175 °C, und einem Druck von 1 bis 35 hPa, bevorzugt von 1 bis 21 hPa, besonders bevorzugt von 2 bis 15 hPa, erhitzt.

Das erfindungsgemäße Verfahren erfolgt bevorzugt kontinuierlich. Dabei wird kontinuierlich in Verdampfungseinheit **1** ein flüssiger Film des Rohprodukts erzeugt, gleichzeitig Fraktion B flüssig über Auslass **31** aus dem Verfahren ausgeschleust, gleichzeitig Gasstrom G erzeugt über Auslass **11** in die Kolonne **4** und von da aus über Leitung **41** in den Kondensator **5** überführt.

Gleichzeitig wird dann Fraktion A in Kondensator **5** und gegebenenfalls Fraktion C in Kondensator **7** kondensiert und gegebenenfalls über Auslass für Fraktion C (72) aus dem Verfahren ausgeschleust. Dabei wird in Kondensator **7** üblicherweise eine Innentemperatur von -10 bis +30 °C, bevorzugt von 0 bis +30 °C, eingestellt. Damit stehen diese unterschiedlichen Prozesse während des erfindungsgemäßen Verfahrens in einem Gleichgewicht. In diesem Gleichgewicht ist die Masse des dem Verfahren zugegebenen Rohprodukts immer im Wesentlichen identisch mit der Summe der Massen an Fraktion A, Fraktion B und Fraktion C, die aus dem Verfahren ausgeschleust werden. "Im Wesentlichen" bedeutet in diesem Zusammenhang, dass die technisch bedingten Schwankungen der Massenströme pro individueller Zeiteinheit damit inbegriffen sind.

In einer besonders bevorzugten Ausführungsform des erfindungsgemäßen Verfahrens wird der Massenstrom an Fraktion B so eingestellt, dass das Massenverhältnis von Fraktion A, die über Leitung **63** aus dem Verfahren geschleust wird, zu Fraktion B, die über Auslass **31** aus dem Verfahren geschleust wird, kontinuierlich von 4 : 1 bis 49 : 1, bevorzugt von 5 : 1 bis 10 : 1 beträgt. Bevorzugt enthält die Fraktion B, die über Auslass **31** aus dem Verfahren geschleust wird, von 40 bis 95 Gew.% Verbindung der Formel (I), sowie von 1,5 bis 50 Gew.% Verbindung der Formal (II).

In einer weiteren besonders bevorzugten Ausführungsform des erfindungsgemäßen Verfahrens wird Fraktion B gesammelt und dann als Rohprodukt erneut in die Verdampfungseinheit 1 gegeben, wobei dann eine Fraktion AA und eine Fraktion BB entsteht. Dadurch wird die chemische Ausbeute an der erfindungsgemäßen Mischung erhöht, ohne dass die Zusammensetzung der erhaltenen erfindungsgemäßen Mischung außerhalb der beanspruchten Grenzen liegt.

Die Erfindung betrifft ebenfalls ein Verfahren zur Herstellung der erfindungsgemäßen Mischungen in den erfindungsgemäßen Vorrichtungen.

Die Erfindung betrifft ebenfalls die nach dem erfindungsgemäßen Verfahren erhältlichen Mischungen die von 98,8 bis 100,0 Gew.%, bevorzugt von 99,0 bis 99,9 Gew.%, Verbindung der Formel (I), und von 0,00 bis 0,70 Gew.%, bevorzugt von 0,0001 bis 0,20 Gew.%, Verbindung der Formel (II) enthalten und die eine Hazen-Farbzahl von 0 bis 15 gemessen nach Methode DIN EN ISO 6271 aufweisen. Bevorzugt enthält die erfindungsgemäße Mischung darüber hinaus von 0,0001 bis 2,0 Gew.% Verbindung der Formel (III). Ein weiterer Vorteil der erfindungsgemäßen Mischungen ist, dass sie einen deutlich geringeren Geruch als die derzeit bekannten Produkte, die mehr als 90 Gew.% Verbindungen der Formel (I) enthalten, aufweisen können. Dies kann in doppelblinden olfaktorischen Untersuchungen eindeutig nachgewiesen werden.

Mit dem erfindungsgemäßen Verfahren lassen sich überraschenderweise die Nachteile der Verfahren des Standes der Technik überwinden und die Mischungen enthaltend der Verbindung der Formel (I) in einer bisher nicht bekannten vorteilhaften Zusammensetzung erhalten. Gleichzeitig weist das erfindungsgemäße Verfahren im Vergleich zu den Verfahren des Standes der Technik vergleichbare oder höhere chemische Ausbeuten der Verbindung der Formel (I) und bei Vermeidung von mehreren extraktiven Aufarbeitungsschritten zur Herstellung des Rohprodukts eine höherer Raum-Zeit-Ausbeute auf, wodurch das erfindungsgemäße Verfahren ökonomischer durchgeführt werden kann. Durch die Vermeidung von mehreren extraktiven Aufarbeitungsschritten kann darüber hinaus die Menge an Abfallstoffen verringert werden, die entweder verbrannt oder anders entsorgt werden müssen. In dem erfindungsgemäßen Verfahren wird in den erfindungsgemäßen Mischungen, also in der isolierten Fraktion A, die Verbindung der Formel (I) in chemischen Ausbeuten von größer als 95 Prozent der Theorie isoliert. Zusätzlich beträgt in dem erfindungsgemäßen Verfahren der Massenanteil an Verbindung der Formel (I), die in Fraktion A und Fraktion B enthalten ist, bezogen auf die Masse an Verbindung der Formel (I), die im Rohprodukt für die Herstellung dieser Fraktion A und B eingesetzt wurde, mehr als 99 Prozent. Damit wird bestätigt, dass in dem erfindungsgemäßen Verfahren nahezu keine Zersetzung der Verbindung der Formel (I) stattfindet.

### Beispiele

### A. Herstellung des Rohprodukts

In einen leeren, inertisierten 2 m³ Hastelloy-C-Reaktor wurden 125 kg 100 %iges Toluol gegeben und nach Beginn des Rührens mit einer Geschwindigkeit von 60 Umdrehungen pro Minute auf 80 °C erwärmt. Anschließend wurden 250 kg Wasser und 481,3 kg (Gehalt: 99 Gew.%, 3,69 kmol) 2-Piperidinethanol (2-(2-Hydroxyethyl)-piperidin) hinzugefügt. Die Zuleitung, über die 2-Piperidinethanol in den Reaktor überführt wurde, wurde mit 50 kg Toluol gespült. Unter Rühren wurden parallel über einen Zeitraum von 6 Stunden 453 kg 32,0 prozentige Natronlauge (3,62 kmol) und 500 kg s-Butylchlorformiat (Gehalt: 99,0 Gew.%, 3,62 kmol) unter Einhaltung eines pH-Wertes von 8,5 bis 9,5 bei 85 °C zugegeben. Die Reaktion ist exotherm und erfordert externe Kühlung, um die Reaktionstemperatur auf 85 °C zu begrenzen.

Nach beendeter Reaktion wurde das Reaktionsgemisch auf 62 °C abgekühlt. 100 kg 10 prozentige Schwefelsäure (0,102 kmol) wurden zugegeben. Das zweiphasige Reaktionsgemisch wurde 15 Minuten nachgerührt. Danach wurde das Rühren beendet, um die Phasentrennung zu ermöglichen. Nach erfolgter Phasentrennung wurden 948 kg untere wässrige Phase aus dem Reaktor abgelassen.

Anschließend wurden für eine erste saure Wäsche zu der im Reaktor verbliebenen organischen Phase 481,2 kg Toluol (Gehalt 100 Gew.%), 40 kg Wasser und 20 kg 10 prozentige Schwefelsäure (20,4 mol) hinzugefügt. Das Gemisch wurde unter Rühren auf 60 °C erwärmt und 15 Minuten nachgerührt. Erneut wurde das Rühren beendet, um die Phasentrennung zu ermöglichen. Nach erfolgter Phasentrennung wurden 57,2 kg untere wässrige Phase aus dem Reaktor abgelassen.

### 1. Rohprodukt A

3.299 g der so erhaltenen organischen Phase wurden durch Zugabe von verdünnter NaOH auf pH 4,8 bis 6 gebracht und die wässrige Phase abgetrennt. Die organische Phase wurde für das erfindungsgemäße Beispiel 4 eingesetzt, nachdem zunächst das darin enthaltenen Toluol bei 90 °C und 80 hPa abdestilliert wurde. Zu den verbliebenden 1.986 g Sumpf wurden 91 g Wasser gegeben und bei 90°C und 60 hPa 145 g abdestilliert, nach erneuter Zugabe von 91 g Wasser und Abdestillation von 97 g Material verblieben 1.877 g Rückstand.

**Das so erhaltene Rohprodukt A wies folgende Zusammensetzung auf:**

| **Komponente** | **Werte** |
|---|---|
| Verbindung der Formel (I) | 97,9 Gew.% |
| Toluol | 0,4 Gew.% |
| Verbindung der Formel (II) | 0,7 Gew.% |
| Verbindung der Formel (III) | 0,05 Gew.% |
| Hazen-Farbzahl | 90 |
| Olfaktorische Bewertung | muffiger Geruch |

### 2. Rohprodukt B

Anschließend wurde die verbliebene organische Phase, von der 3.299 g für die Herstellung des Rohprodukts A abgenommen wurde, für die zweite saure Wäsche abermals mit 40,0 kg Wasser und 20,0 kg 10 prozentiger Schwefelsäure (20,4 mol) versetzt. Das Gemisch wurde 15 Minuten nachgerührt. Erneut wurde das Rühren beendet, um die Phasentrennung zu ermöglichen. Nach erfolgter Phasentrennung wurden 58,5 kg untere wässrige Phase aus dem Reaktor abgelassen.

Zur Neutralisation wurde eine dritte Wäsche angeschlossen, indem zu der im Reaktor verbliebenen organischen Phase 125 kg Wasser gegeben wurde, um dann die Reaktionsmischung durch Zugabe von 0,13 kg 32 %iger Natronlauge (1 mol) auf einen pH-Wert von 4,8 bis 5,0 zu bringen. Das Gemisch wurde 10 Minuten nachgerührt. Erneut wurde das Rühren beendet, um die Phasentrennung zu ermöglichen. Nach erfolgter Phasentrennung wurden 140,3 kg untere wässrige Phase aus dem Reaktor abgelassen.

Als letzte Wäsche wurde die im Reaktor verbliebenen organischen Phase mit 125 kg Wasser durchgeführt, wobei der pH-Wert des Gemisches mit 0,13 kg 32 %iger Natronlauge (1 mol) auf einen Bereich von 4,8 bis 7,0 eingestellt wurde. Das Gemisch wurde 10 Minuten nachgerührt. Erneut wurde das Rühren beendet, um die Phasentrennung zu ermöglichen. Nach erfolgter Phasentrennung wurden 137,5 kg untere wässrige Phase abgelassen.

Von der verbliebenen organischen Phase wurden 641,3 kg des Lösungsmittels Toluol bei einem Druck von 650 hPa und einer Temperatur des Sumpfes von bis zu 90 °C abdestilliert. Anschließend wurden 37,5 kg Wasser zugegeben und es wurde 60 Minuten nachgerührt. Dann wurden von dem Sumpf 40,0 kg Wasser bei maximal 90 °C und 60 hPa abdestilliert. Anschließend wurde der Druck im Reaktor mit Stickstoff auf Umgebungsdruck erhöht, auf eine Temperatur von kleiner als 60 °C abgekühlt und das Rohgemisch aus dem Reaktor über einen Filter abgelassen.

Es wurden 825,0 kg Rohgemisch erhalten, das als Ausgangsprodukt für das erfindungsgemäße Verfahren verwendet wurde.

**Das so erhaltene Rohprodukt B wies folgende Zusammensetzung auf:**

| **Komponente** | **Werte** |
|---|---|
| Verbindung der Formel (I) | 98,5 Gew.% |
| Toluol | 0,03 Gew.% |
| Verbindung der Formel (II) | 0,8 Gew.% |
| Verbindung der Formel (III) | 0,1 Gew.% |
| Hazen-Farbzahl | 40 |
| Olfaktorische Bewertung | Leicht muffiger Geruch |

### B. Verfahren zur Herstellung von Mischungen aus dem Rohprodukt

### Beispiel 1 (nicht erfindungsgemäß)

In einer Apparatur bestehend aus einem 2 L-Planschlifftopf mit 10 bödiger Kolonne, Dephlegmator mit Rückflussteiler und Vakuumpumpe mit Kühlfalle wurden 1.523 g Rohprodukt B vorgelegt und die Anlage auf 20 hPa evakuiert. Der Dephlegmator wurde auf 50°C und der Rücklaufteiler auf ein Rücklaufverhältnis (Volumenverhältnis von Rücklauf zu Destillatentnahme) von 1 : 1 eingestellt wurde. Bei ca. 175°C Innentemperatur siedete das System, jedoch kondensierte das allermeiste Material in der Kühlfalle. Die Destillation wurde daraufhin abgebrochen. Ca. 102 g des Materials befanden sich in der Kühlfalle und ca. 1.386 g im Sumpf. Das Material in der Kühlfalle bestand zu ca. 99% aus 2-Butanol, der Sumpf im Planschlifftopf zu ca. 82,3 Gew. % aus Verbindung der Formel (I) und zu 16 % aus Verbindung der Formel (III), das sich durch Abspaltung von Butanol aus Verbindung der Formel (I) bildet. Damit ließ sich bei 175 °C und 20 hPa keine Verbindung der Formel (I) als Destillat erhalten. Vielmehr erfolgte eine Zersetzung des Produkts durch Butanolabspaltung. Die Verweilzeit der flüssigen Phase enthaltend ein Rohprodukt betrug dabei mehr als 60 Minuten.

### Beispiel 2 (erfindungsgemäß)

Die für dieses Beispiel verwendete Apparatur bestand aus einem Dünnschichtverdampfer mit Ölmantel (30 cm lang, Innendurchmesser 5 cm), 3 Wischerarmen mit Rollen und einer Innentemperaturmessung am Sumpfausgang und einem thermostatisierten Kondensator (Dephlegmator), Vakuumpumpe mit Kühlfalle. Der Verdampfer wurde mit 150 bis 160°C heißem Öl aufgeheizt und das Vakuum auf 2 hPa eingestellt. Der Kondensator wurde mit 50 °C warmem Öl betrieben. 200 bis 250 g Rohprodukt B wurden pro Stunde kontinuierlich eindosiert. Die Wischerdrehzahl betrug 120 U/min. Dabei betrug die Innentemperatur des Dünnschichters zwischen 135 und 140°C an. Insgesamt wurden so 5.107 g Saltidin dosiert. Dabei fielen 4.869 g farbloses Destillat als Fraktion A an, 130 g brauner flüssiger Sumpf verließen den Dünnschichter als Fraktion B und 7,1 g Flüssigkeit wurden in der Kühlfalle als Fraktion C aufgefangen.. Dies entspricht einer Massenbilanz von 98 %. Die mittlere Verweilzeit der flüssigen Phase enthaltend ein Rohprodukt und/oder Fraktion B oder deren beliebigen Mischungen betrug dabei weniger als 60 Sekunden. Das so erhaltene Produkt hatte folgende Eigenschaften:

| **Komponente** | **Werte** |
|---|---|
| Verbindung der Formel (I) | 98,9 Gew.% |
| Toluol | nn |
| Verbindung der Formel (II) | 0,7 Gew. % |
| Verbindung der Formel (III) | 0,12 Gew.% |
| Hazen-Farbzahl | 5 bis 12 |
| Olfaktorische Bewertung | wenig bis gar nicht riechend |
| Ausbeute der Theorie für Fraktion A | >95 % |

In der Kühlfalle wurde ein Kondensat (kondensierte Fraktion C) aufgefangen, das 97 Gew.% Wasser enthielt. Der braune bis dunkelbraune Sumpf enthielt ca. 200 bis 500 ppm Natriumchlorid und Natriumsulfat, neben weiteren Spuren anorganischer Bestandteile.

### Beispiel 3 (erfindungsgemäß)

Die für dieses Beispiel verwendete Apparatur bestand aus einem Dünnschichtverdampfer mit Ölmantel (30 cm lang, Innendurchmesser 5 cm), 3 Wischerarmen mit Rollen und einer Innentemperaturmessung am Sumpfausgang, einer 10 bödigen verspiegelten begleitbeheizten Kolonne, die mit einer mit Packung von HC4 Maschendrahtringen (Packung 30 cm lang, Durchmesser 24 mm, ca. 10 theoretische Böden) gefüllt ist, einem Rückflussteiler und einem thermostatisierten Kondensator (Dephlegmator), Vakuumpumpe mit Kühlfalle. Der Verdampfer wurde mit 178 °C heißem Öl aufgeheizt und das Vakuum auf 3 hPa eingestellt. Die isolierte Kolonne wurde zur thermischen Isolation außen mit 164° C begleitbeheizt. Der Kondensator wurde mit 90 °C warmem Öl betrieben. 60 g Rohprodukt B wurden pro Stunde kontinuierlich zwischen Kolonne und Dünnschichtverdampfer eindosiert, wobei über den Rückflussteiler ein Rücklaufverhältnis von 1 : 2 (Rücklauf / Entnahme, Volumen / Volumen) eingestellt wurde. Die Wischerdrehzahl betrug 120 U/min. Dabei betrug die Innentemperatur des Dünnschichters am unteren Ende zwischen 155 und 160°C. Am Sumpf stellte sich ein Druck von 5,5 hPa ein, während am Kolonnenkopf ein Druck von 3,3 hPa gemessen wurde. Insgesamt wurden so 1715 g Rohprodukt B dem Dünnschichtverdampfer zudosiert. Dabei fielen 1687 g Fraktion A als farbloses Destillat an, 17 g Fraktion B in Form eines braunen flüssigen Sumpfes verließen den Dünnschichter und 3,8 g Fraktion C wurden in der Kühlfalle kondensiert. Dies entspricht einer Massenbilanz von > 99 Gew.%. Die mittlere Verweilzeit der flüssigen Phase enthaltend ein Rohprodukt und/oder Fraktion B oder deren beliebigen Mischungen betrug dabei weniger als 60 Sekunden. Das so erhaltene Produkt hatte folgende Eigenschaften:

| **Komponente** | **Werte** |
|---|---|
| Verbindung der Formel (I) | 99,6 Gew.% |
| Toluol | <0,05 Gew.% |
| Verbindung der Formel (II) | <0,005 Gew.% |
| Verbindung der Formel (III) | 0,13 Gew.% |
| Hazen-Farbzahl | <10 |
| Olfaktorische Bewertung | Wenig bis gar nicht riechend |
| Ausbeute der Theorie für Fraktion A | >95 % |

In der Kühlfalle wurde ein Kondensat (kondensierte Fraktion C) aufgefangen, das 90 Gew.% 2-Butanol, 1,7 Gew.% Toluol, 1,1 Gew.% Saltidin sowie weitere Bestandteile enthielt. Der braune bis dunkelbraune Sumpf enthielt 76 Gew.% Saltidin, 20,1 Gew.% Verbindung der Formel (II) sowie weitere Bestandteile.

### Beispiel 4 (erfindungsgemäß)

Die für dieses Beispiel verwendete Apparatur bestand aus einem Dünnschichtverdampfer mit Ölmantel (30 cm lang, Innendurchmesser 5 cm), 3 Wischerarmen mit Rollen und einer Innentemperaturmessung am Sumpfausgang, einer 10 bödigen verspiegelten begleitbeheizten Kolonne, die mit einer mit Packung von HC4 Maschendrahtringen (Packung 30 cm lang, Durchmesser 24 mm, ca. 10 theoretische Böden) gefüllt ist, einem Rückflussteiler und einem thermostatisierten Kondensator (Dephlegmator), Vakuumpumpe mit Kühlfalle. Der Verdampfer wurde mit 178 °C heißem Öl aufgeheizt und das Vakuum auf 3 hPa eingestellt. Die isolierte Kolonne wurde zur thermischen Isolation außen mit 164° C begleitbeheizt. Der Kondensator wurde mit 90 °C warmem Öl betrieben. 70 g Rohprodukt A wurden pro Stunde kontinuierlich zwischen Kolonne und Dünnschichtverdampfer eindosiert, wobei über den Rückflussteiler ein Rücklaufverhältnis von 1 : 2 (Rücklauf / Entnahme, Volumen / Volumen) eingestellt wurde. Die Wischerdrehzahl betrug 200 U/min. Dabei betrug die Innentemperatur des Dünnschichters am unteren Ende zwischen 155 und 160°C. Am Sumpf stellte sich ein Druck von 7 hPa, während am Kolonnenkopf ein Druck von 3,0 hPa gemessen wurde. Insgesamt wurden so 1.365 g Saltidin dosiert. Dabei fielen 1.300 g farbloses Destillat an, 55 g brauner flüssiger Sumpf verließen den Dünnschichter und 2 g Flüssigkeit wurden in der Kühlfalle aufgefangen. Dies entspricht einer Massenbilanz von >99 %. Die mittlere Verweilzeit der flüssigen Phase enthaltend ein Rohprodukt und/oder Fraktion B oder deren beliebigen Mischungen betrug dabei weniger als 60 Sekunden. Das so erhaltene Produkt hatte folgende Eigenschaften:

| **Komponente** | **Werte** |
|---|---|
| Icaridin | 99,5 Gew.% |
| Toluol | <0,05 Gew.% |
| Verbindung der Formel (II) | <0,005 Gew.% |
| Verbindung der Formel (III) | 0,07 Gew.% |
| Farbzahl | <10 |
| Olfaktorische Bewertung | Wenig bis gar nicht riechend |
| Ausbeute der Theorie für Fraktion A | > 95% |

In der Kühlfalle wurde ein Kondensat (kondensierte Fraktion C) aufgefangen, das 90 Gew.% 2-Butanol, 1,7 Gew.% Toluol, 1,1 Gew.% Saltidin sowie weitere Bestandteile enthielt. Der braune bis dunkelbraune Sumpf enthielt 76 Gew.% Saltidin, 20,1 Gew.% Verbindung der Formel (II) sowie weitere Bestandteile.

## Patentansprüche

1. Mischung enthaltend als Komponenten von 98,8 bis 100,0 Gew.%, bevorzugt von 99,0 bis 99,9 Gew.%, Verbindung der Formel (I), 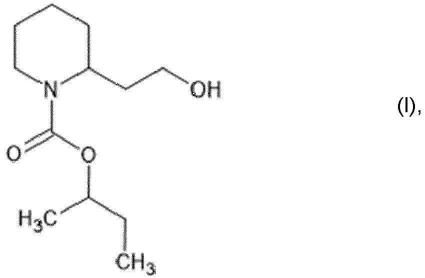 von 0,00 bis 0,70 Gew.%, bevorzugt von 0,0001 bis 0,20 Gew.%, Verbindung der Formel (II), 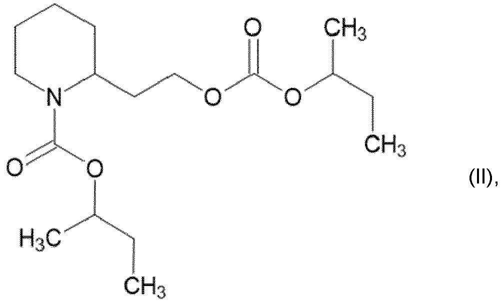 sowie weitere Bestandteile, wobei sich die Gewichtsprozente der Komponenten zu 100 Gew.% addieren,
wobei die Mischung eine Hazen-Farbzahl von 0 bis 15 gemessen nach Methode DIN EN ISO 6271 aufweist.

2. Mischung nach Anspruch 1, die von 0,0001 bis 2,0 Gew.% Verbindung der Formel (III), 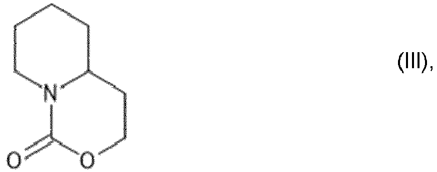 enthält.

3. Verfahren zur Herstellung der Mischung nach Anspruch 1 oder 2 ausgehend von einen Rohprodukt enthaltend
∘ von 94,0 bis 99,0 Gew.% Verbindung der Formel (I) und
∘ von 0,50 bis 5,0 Gew.% Verbindung der Formel (II),
wobei zumindest eine Fraktion A (Destillat) von einer Fraktion B (Sumpf) und einer gasförmigen Fraktion C abgetrennt wird, indem
a) das Rohprodukt als flüssige Phase derart, bevorzugt durch den Kontakt mit einer festen erhitzten Oberfläche, thermisch behandelt wird, sodass ein Gasstrom G enthaltend Fraktion A und Fraktion C erzeugt wird, und die Fraktion B als flüssige Phase verbleibt, und
b) gleichzeitig und / oder nachfolgend Fraktion B flüssig aus dem Verfahren ausgeschleust wird, und
c) gleichzeitig und / oder nachfolgend Fraktion A aus Gasstrom G kondensiert und aus dem Verfahren ausgeschleust wird, wobei die Mischung gemäß Anspruch 1 oder 2 erhalten wird, und
d) gegebenenfalls gleichzeitig und / oder nachfolgend die Fraktion C kondensiert und aus dem Verfahren ausgeschleust wird,
wobei die Verweilzeit der flüssigen Phase während der thermischen Behandlung, bevorzugt die Verweilzeit der flüssigen Phase enthaltend ein Rohprodukt und/oder Fraktion B oder deren beliebigen Mischungen, von 1 bis 900 Sekunden, bevorzugt von 10 bis 600 Sekunden, besonders bevorzugt von 10 bis 300 Sekunden, beträgt.

4. Verfahren zur Herstellung der Mischung nach Anspruch 3 ausgehend von einen Rohprodukt, das eine Hazen-Farbzahl von mindestens 40 gemessen nach Methode DIN-ISO 6271 aufweist.

5. Verfahren nach Anspruch 3 oder 4, **dadurch gekennzeichnet, dass** es in einer Vorrichtung durchgeführt wird, die zumindest
• eine Verdampfungseinheit (evaporation unit) (1), Einlass für Rohprodukt (21) und Auslass für Fraktion B (31), umfasst,
o wobei die Verdampfungseinheit (evaporation unit) (1) zumindest
∘ einen beheizbaren Gehäusemantel, welcher einen sich in axialer Richtung erstreckenden rotationssymmetrischen Verdampfungsraum (evaporation space) umschließt, und
∘ eine in dem Verdampfungsraum angeordnete und sich koaxial erstreckende, antreibbare Rotorwelle zur Erzeugung eines Rohproduktfilms auf der Innenfläche des Gehäusemantels und zur Förderung des Materials in Richtung vom Einlass für Rohprodukt (21) zum Auslass für Fraktion B (31) hin, wobei die Rotorwelle einen zentralen Rotorwellenkörper und auf dessen Umfang angeordnete Rotorelemente umfasst, deren radial äußerstes Ende einen Abstand zur Innenfläche des Gehäusemantels aufweist,
• Kondensator (5), Rückflussteiler (6), und Auslass für Fraktion A (63), und bevorzugt Kolonne (4), umfasst,
∘ wobei in Schritt a) das Rohprodukt derart thermisch behandelt wird, dass das Rohprodukt über den Einlass (21) in den Verdampfungsraum der Verdampfungseinheit (1) eingetragen wird und auf der Innenseite des Gehäusemantels ein flüssiger Film des Rohproduktes erzeugt wird, wodurch der flüssige Film des Rohproduktes auf eine Temperatur erhitzt wird, sodass zumindest ein Teil der Fraktion A und der Fraktion C in den gasförmigen Zustand überführt und als Gasstrom G aus der Verdampfungseinheit (1), bevorzugt über den Auslass (11), ausgetragen wird, wobei das Verhältnis der Massenströme zwischen Fraktion B und Gasstrom G von 1 : 20 bis 1 : 5 beträgt, und
∘ in Schritt b) die Fraktion B über den Auslass (31) aus der Verdampfungseinheit (1) ausgetragen wird, und
∘ in Schritt c) Gasstrom G aus der Verdampfungseinheit (1), bevorzugt über Kolonne (4), in den Kondensator (5) überführt wird, wobei Fraktion A kondensiert und über den Rückflussteiler (6) und Auslass (63) erhalten wird.

6. Verfahren nach Anspruch 5, **dadurch gekennzeichnet, dass** es in einer Vorrichtung durchgeführt wird, die einen Kondensator (7) umfasst, in dem in Schritt d) Fraktion C kondensiert wird.

7. Verfahren nach Anspruch 5 oder 6, **dadurch gekennzeichnet, dass** der flüssige Film, der in der Verdampfungseinheit (1) thermisch behandelt wird, eine Filmdicke von 0,001 bis 2 mm, bevorzugt von 0,005 bis 1 mm aufweist.

8. Verfahren nach einem der Ansprüche 3 bis 7, wobei in Schritt a) das Überführen der Fraktion A in die Gasphase
• bei einer Temperatur von 120 bis 200 °C, bevorzugt von 120 bis 185 °C, besonders bevorzugt von 120 bis 175 °C und einem Druck von 1 bis 35 hPa, bevorzugt von 1 bis 21 hPa, besonders bevorzugt von 2 bis 15 hPa, oder
• bei einer Temperatur erfolgt, die maximal 40 °C, bevorzugt maximal 30 °C, über dem Siedepunkt der Fraktion A liegt,
erfolgt.

9. Verfahren zur Herstellung nach einem der Ansprüche 3 bis 8, **dadurch gekennzeichnet, dass** dem Rohprodukt zu keinem Zeitpunkt des Verfahrens ein Schiebemittel zugefügt wird.

10. Verfahren nach Anspruch 3 bis 9, wobei das Rohprodukt mit den Schritten umfassend
i. Umsetzung von 2-Hydroxyethylpiperidin mit Chlorameisensäure-sek.-butylester in Gegenwart oder Abwesenheit zumindest einer Base und in Gegenwart oder Abwesenheit zumindest eines Lösungsmittels, wobei ein Reaktionsgemisch erhalten wird, das von 35 bis 65 Gew.% Verbindung der Formel (I) enthält, und
ii. nachfolgend, gegebenenfalls unter Vermischung, Zugabe von Säure und/oder Wasser zu dem Reaktionsgemisch aus Schritt i., wobei ein zweiphasiges Reaktionsgemisch umfassend eine organische und eine wässrige Phase entsteht, und
iii. nachfolgend gegebenenfalls Trennung der organischen Phase von der wässrigen Phase des Reaktionsgemisches aus Schritt ii.
iv. nachfolgend gegebenenfalls Wäsche der organischen Phase aus Schritt iii. mit wässriger Säure
v. nachfolgend gegebenenfalls Trocknung der organischen Phase aus Schritt iv.
vi. nachfolgend gegebenenfalls Abtrennung des zumindest einen Lösungsmittels von der organischen Phase aus Schritt v., wobei das Rohprodukt erhalten wird.

11. Vorrichtung umfassend zumindest eine Verdampfungseinheit (1), Behälter für Fraktion B (3), einen Kondensator (5) und eine Pumpe (8).

12. Vorrichtung nach Anspruch 11, umfassend zusätzlich eine Kolonne (4), eine kommunizierende Leitung (11) zwischen Verdampfungseinheit (1) und Kolonne (4), einen Rückflussteiler (6), und einen Kondensator (7).

13. Vorrichtung nach Anspruch 12, umfassend zusätzlich Einlass für Rohprodukt (21), kommunizierende Leitung (41) zwischen Kolonne (4) und (5), kommunizierende Leitung (51) zwischen Kondensator (5) und Kondensator (7), kommunizierende Leitung (61) zwischen Kondensator (5) und Rückflussteiler (6), kommunizierende Leitung (62) zwischen Kolonne (4) und Rückflussteiler (6), Auslass für Fraktion A (63) und Auslass für Fraktion C (72).

14. Verfahren nach einem der Ansprüche 3 bis 10 zur Herstellung der Mischung nach Anspruch 1 oder 2 in einer Vorrichtung nach einem der Ansprüche 11 bis 13.
